# EUROPEAN PATENT APPLICATION

(11) **EP 4 609 856 A1**
(43) Date of publication of application: **03.09.2025**
(21) Application number: 23883110.1
(22) Date of filing: 26.10.2023
(51) Int. Cl.: A61K 9/00, A61K 9/14, A61K 31/465

(54) **DRY POWDER INHALER STICK CONTAINING COUGH SUPPRESSANT AND DRY POWDER INHALER COMPRISING SAME**

(30) Priority: 27.10.2022 KR 20220140558; 13.10.2023 KR 20230136886
(71) Applicant: KT&G Corporation, Daedeok-gu Daejeon 34337 (KR)
(72) Inventor: SEO, Man Seok, Daejeon 34128 (KR); KIM, Moonwon, Daejeon 34128 (KR); JUNG, Yongmi, Daejeon 34128 (KR); SHIN, Jun Won, Daejeon 34128 (KR)
(74) Representative: Ter Meer Steinmeister & Partner
(86) International application number: PCT/KR2023/016698
(87) International publication number: WO 2024/091005

(57) **Abstract**

The present invention provides a dry powder inhaler stick and a dry powder inhaler comprising same, wherein the dry powder inhaler stick comprises a filter portion, a capsule portion, and a tube portion, the capsule portion comprises dry nicotine powder, and at least one of the filter portion and the tube portion comprises a cough suppressant.

## Description

### TECHNICAL FIELD

The present disclosure relates to a stick for a dry powder inhaler (DPI) containing a cough suppressant and a DPI including the same.

### BACKGROUND ART

Nicotine is delivered into the body by various methods, such as smoking combustible tobacco such as cigarettes, cigars, or pipes, using a method of generating an aerosol by heating an aerosol-generating substance in a cigarette, or directly inhaling a nicotine powder or the like.

Among these, the method of directly inhaling the nicotine powder uses a dry powder inhaler (DPI) to deliver the dry powder directly into the user's respiratory tract by inhalation. In addition to nicotine, the dry powder is also used to contain various effective ingredients (drugs) for the purpose of improving diseases, and the like.

DPI tobacco is inhalable tobacco product that uses a medical inhalation technology to provide smokers with a feeling of satisfaction by inhaling a powder containing nicotine (1% or less by weight). The inhalable tobacco product may be a stick containing a powder (nicotine and a powder material for nicotine transfer) and a capsule containing the powder, and a device for implementing smoking.

Meanwhile, when inhaling the DPI tobacco, consumers may cough as the powder is transferred to the respiratory tract, which may be a factor that reduces the satisfaction of inhalation for consumers.

Menthol or the like is used to suppress or reduce the cough, however, when the process of manufacturing and drying a nicotine powder using menthol is carried out, there is a concern that problems such as a decrease in a yield of nicotine or the like, uniformity of powder sizes, and storage stability may occur.

### DISCLOSURE OF THE INVENTION

### TECHNICAL GOALS

One object of the present disclosure is to solve the above problems by applying menthol to filter components forming a stick for a DPI, rather than applying menthol as a cough suppressant when manufacturing a DPI powder in the related art, thereby suppressing cough and reducing quality deterioration and process problems occurring in the nicotine-containing powder manufacturing process.

However, goals to be achieved are not limited to those described above, and other goals not mentioned above are clearly understood by one of ordinary skill in the art from the following description.

### TECHNICAL SOLUTIONS

According to one embodiment of the present disclosure, there is provided a stick for a dry powder inhaler (DPI), the stick including a filter portion, a capsule portion, and a tube portion,
wherein the capsule portion includes a nicotine dry powder, and
a cough suppressant is included in one or more of the filter portion and the tube portion.

According to another embodiment of the present disclosure, a DPI including the stick for a DPI described above.

### EFFECTS OF THE INVENTION

A stick for a dry powder inhaler (DPI) containing a cough suppressant and a DPI including the same according to one aspect of the present disclosure have advantages of being able to effectively suppressing cough and reducing quality deterioration and process problems occurring in the nicotine-containing powder manufacturing process.

### BEST MODE FOR CARRYING OUT THE INVENTION

Hereinafter, embodiments will be described in detail with reference to the accompanying drawings. However, various alterations and modifications may be made to the embodiments. Here, the embodiments are not construed as limited to the disclosure. The embodiments should be understood to include all modifications, equivalents, and substitutions within the scope and spirit of the present disclosure.

The terminology used herein is for the purpose of describing particular embodiments only and is not to be limiting of the embodiments. As used herein, the singular form is intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which the embodiments belong. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

When describing the embodiments with reference to the accompanying drawings, the same or similar elements are denoted by the same reference numerals even though they are depicted in different drawings, and redundant descriptions thereof will be omitted. In the following description of the embodiments, a detailed description of known functions and configurations incorporated herein will be omitted when the same may make the subject matter of the embodiments disclosed in the present specification rather unclear.

In addition, the terms first, second, A, B, (a), and (b) may be used to describe constituent elements of the embodiments. These terms are used only for the purpose of discriminating one component from another component, and the nature, the sequences, or the orders of the components are not limited by the terms.

A component, which has the same common function as a component included in any one embodiment, will be described by using the same name in other embodiments. Unless otherwise mentioned, the descriptions of one embodiment may be applicable to other embodiments and thus, repeated descriptions will be omitted for conciseness.

The term "dry powder inhaler (DPI)" used herein refers to a device that delivers substances produced by a reaction between a nicotine-related substance and an organic acid substance into the user's lungs through the user's oral cavity.

According to one embodiment of the present disclosure, there is provided a stick for a DPI including a filter portion, a capsule portion, and a tube portion,
wherein the capsule portion includes a nicotine dry powder, and
a cough suppressant is included in one or more of the filter portion and the tube portion.

Each component of the stick for the DPI is specifically described as follows.

### [Capsule portion]

The capsule portion included in the stick for the DPI may include a nicotine dry powder and may not separately include a cough suppressant. The nicotine dry powder may be a substance containing nicotine or a nicotine salt.

Here, the nicotine salt may be present in the form of a salt of nicotine and organic acid, and the organic acid may correspond to, but is not limited to, lactic acid, tartaric acid, pyruvic acid, and/or salicylic acid.

When forming the nicotine salt with the organic acid as described above, the nicotine salt may be in the form of, for example, nicotine lactate, nicotine tartrate, nicotine pyruvate, or nicotine salicylate. Among these, considering the stability and harmfulness to the human body and the like, since the nicotine dry powder is used for human inhalation, the organic acid is desirably lactic acid, and the nicotine salt is desirably nicotine lactate accordingly.

The nicotine dry powder may contain amino acid and an excipient, in addition to the nicotine or the nicotine salt.

The type of amino acid is not particularly limited, but may include one or more of leucine, lysine, valine, arginine, threonine, and/or methionine, and the amino acid may desirably include leucine.

As described above, when leucine is included as the amino acid, flowability and a transfer amount of a finally formed nicotine dry powder may be improved, unlike in a case of other amino acids.

In addition, it is desirable that the content of amino acid included at this time is 5 wt% or less based on a total content of the dry powder. By controlling the content of amino acid to 5 wt% or less as described above, a particle size and particle size distribution of the finally formed nicotine dry powder may be improved.

The type of excipient included in the nicotine dry powder may include sugar and/or sugar alcohol, and the type of sugar may include sucrose, trehalose, dextrose, glucose, maltose, and the like. Furthermore, the type of sugar alcohol may include mannitol, sorbitol, galactitol, maltitol, xylitol, lactitol, and the like.

Among these, it is desirable that the excipient include mannitol, which has a shape close to a sphere, in terms of ensuring additive safety and the flowability of the powder.

The nicotine dry powder is manufactured specifically through a spray drying process using the nicotine or the nicotine salt, the amino acid, and the excipient described in detail above. When nicotine dry powder is manufactured using the spray drying process, better results are obtained in terms of yield than when jet milling is used. This is understood to be because, when jet milling or the like is used, nicotine evaporates into the air due to the characteristics of the nicotine dry powder.

Meanwhile, when performing the spray drying, a solvent, the nicotine or nicotine salt, the amino acid, and the excipient may be stirred first using a magnetic stirrer, and then the spray drying may be performed. At this time, water is used as the solvent, and the spray drying is performed at an inlet temperature (inlet temp.) of about 100°C at a specific spray flow rate and atmosphere flow rate to form a nicotine dry powder.

The spray flow rate is a factor that determines a final yield, a total spray time, properties, and the like of the nicotine dry powder, and may be appropriately 2 to 7 mL/min. The atmosphere flow rate thereof may be appropriately 0.3 to 0.4 m3/min, considering the final yield and the particle size of the nicotine dry powder. In addition, the flow rate of an aspirator is determined by pressure or a flow rate depending on the characteristics of the equipment, and may be appropriately about 250 kPa or 1200 L/hr.

As described above, unlike DPI cigarettes of the related art, the capsule portion according to one embodiment of the present disclosure does not include a separate cough suppressant such as menthol during the manufacturing of the nicotine dry powder (spray drying process). Accordingly, there is no need to maintain the temperature at 80°C or higher during the manufacturing process, and when manufacturing the nicotine dry powder including a cough suppressant such as menthol, problems in the manufacturing process such as a reduced yield, uniformity of powder sizes, and storage stability may not occur.

### [Filter portion]

The filter portion included in the stick for the DPI may be made of a cellulose acetate filter including acetate tow, a paper filter, or a filter made of a polymer material. The filter portion may be formed of one segment, or may be formed by being divided into a plurality of segments, such as a first filter portion, a second filter portion, and the like.

According to one embodiment of the present disclosure, the filter portion may include a cough suppressant, and examples of the cough suppressant may include menthol, mint, saccharin, benzocaine, and the like, and may desirably include menthol.

For example, when forming a cellulose acetate filter containing acetate tow using menthol as the cough suppressant, the menthol may be contained in a capsule and positioned inside the acetate tow, or the acetate tow may be flavored with a Transport Jet Nozzle System (TJNS).

Meanwhile, when the filter portion is divided into a plurality of segments, the flavored filter or the filter including the capsule containing the cough suppressant may be included in at least one of the plurality of segments.

### [Tube portion]

The tube portion included in the stick for the DPI is a portion formed by applying steam pressure or the like into a tube shape containing acetate tow, and may contain a flavoring material by applying an acetate tow material. When a consumer smokes, the flavoring material may move to the oral cavity, allowing the consumer to feel the characteristics and taste of the flavor. In addition, as described above for the filter portion, a cough suppressant may be included.

In this case, the type of the cough suppressant is substantially the same as the cough suppressant of the filter portion described in detail above, and the cough suppressant such as menthol may be sprayed inside or outside the tube portion.

The cough suppressant, such as menthol, may be included alternatively in the filter portion or the tube portion, or may be positioned in both the filter portion and the tube portion.

As described above, by positioning the cough suppressant in the filter portion and/or the tube portion separately from the nicotine dry powder positioned in the capsule portion, there is no need to manufacture a powder containing the cough suppressant such as menthol simultaneously with the nicotine dry powder, and compared to manufacturing a final powder by including the nicotine dry powder and the cough suppressant such as menthol, the content of menthol applied to the acetate tow may be relatively accurately and easily controlled, and it may be seen that there are advantages such as the yield, the uniformity of powder sizes, and the storage stability.

Meanwhile, according to another embodiment of the present disclosure, there is provided a DPI including the stick for the DPI.

In this case, the stick for the DPI included in the DPI may be considered to be substantially the same as that described in detail above.

Hereinafter, the configuration of the present disclosure and effects thereof will be described in more detail through examples and comparative examples. However, the examples are merely intended for the purpose of describing the disclosure in more detail, and thus, the scope of the disclosure is not limited to the examples.

### <Examples>

### 1. Manufacturing Example 1

### (1) Manufacturing of nicotine dry powder - Spray drying

The materials were weighed according to the composition ratio in Table 1 below, placed in DI water, and stirred for about 10 minutes, and a nicotine dry powder was manufactured through a spray drying process.

**[Table 1]**

| | Weight (g) | Weight (%) |
|---|---|---|
| Nicotine | 0.16 | 0.8 |
| Lactic acid | 0.8 | 4 |
| Leucine | 1.66 | 8.3 |
| Mannitol | 17.38 | 86.9 |
| Total | 20 | 100 |

### (2) Manufacturing of capsules containing nicotine dry powder

30 mg of the manufactured nicotine dry powder was weighed and manually placed into a capsule for inhalation to manufacture a capsule.

### 2. Manufacturing Example 2

As shown in Table 2 below, menthol, the cough suppressant, was applied to a stick for a DPI including a filter portion formed of acetate tow, a capsule portion, and a tube portion to manufacture the stick for the DPI of the present disclosure.

When applying menthol to the filter portion, menthol was flavor-sprayed by the TJNS method or a capsule containing menthol was applied, and when applying menthol to the tube portion, menthol was applied to the inside of a tube and a molded tube was manufactured.

**[Table 2]**

| Classification | Filter portion | Capsule portion | Tube portion |
|---|---|---|---|
| Length (mm) | 12 | 20 | 16 |
| Applied material | Acetate tow (9.0Y/25k) | capsule + powder | Acetate tow molding |
| Example 1 | tow | capsule + nicotine dry powder | Tube shape molding (containing menthol) |
| Example 2 | Tow + menthol capsule | capsule + nicotine dry powder | Tube shape molding (except for menthol) |
| Example 3 | Tow + menthol flavoring | capsule + nicotine dry powder | Tube shape molding (except for menthol) |
| Example 4 | Tow + menthol flavoring | capsule + nicotine dry powder | Tube shape molding (containing menthol) |
| Example 5 | Tow + menthol capsule | capsule + nicotine dry powder | Tube shape molding (containing menthol) |

### 3. Test Example: Measurement of particle size, particle size distribution, and transfer characteristics

Mass median aerodynamic diameters (MMAD), fine particle fractions (FPF), and transfer amounts were compared between cases where only the nicotine dry powder was manufactured and cases where the nicotine dry powder and flavoring powder were mixed.

**[Table 3]**

| | MMAD (µm) | FPF (%) | Amount of transfer (based on 30mg in total) |
|---|---|---|---|
| Nicotine dry powder (0.99 wt%) | 3.911 | 48.47 | 25.9 |
| Nicotine dry powder 0.99 + flavoring powder | 4.686 | 38.15 | 9.8 |
| Nicotine dry powder (0.75 wt%) | 3.923 | 48.9 | 25.9 |
| Nicotine dry powder 0.75 + flavoring powder | 4.834 | 36.12 | 2.1 |
| Nicotine dry powder (0.5 wt%) | 4.138 | 48.71 | 24.3 |
| Nicotine dry powder 0.5 + flavoring powder | 4.563 | 42.22 | 3.1 |

As shown in Table 3 above, it may be confirmed that, when only the nicotine dry powder is manufactured, the MMAD value is lower, the FPF value is closer to 50%, and the transfer amount is also excellent, compared to when the powder is manufactured by mixing the nicotine dry powder and the flavoring powder.

Through this, it may be seen that, when only the nicotine dry powder is applied to the capsule separately, the inhalation efficiency of nicotine and the like is excellent for the user compared to when both the nicotine dry powder and the flavoring powder are applied to the capsule. It may be seen that it is more desirable to apply menthol, the cough suppressant, to the filter portion or tube portion rather than introducing menthol to the capsule portion.

While the embodiments are described with reference to drawings, it will be apparent to one of ordinary skill in the art that various alterations and modifications in form and details may be made in these embodiments without departing from the spirit and scope of the claims and their equivalents. For example, suitable results may be achieved if the described techniques are performed in a different order, and/or if components in a described system, architecture, device, or circuit are combined in a different manner, and/or replaced or supplemented by other components or their equivalents.

Therefore, other implementations, other embodiments, and equivalents to the claims are also within the scope of the following claims.

## Claims

1. A stick for a dry powder inhaler (DPI), the stick comprising:
a filter portion,
a capsule portion, and
a tube portion,
wherein the capsule portion comprises a nicotine dry powder, and
a cough suppressant is included in one or more of the filter portion and the tube portion.

2. The stick of claim 1, wherein the cough suppressant comprises one or more of menthol, mint, saccharin, and benzocaine.

3. The stick of claim 1, wherein the filter portion comprises an acetate toe, and the cough suppressant is flavored on the acetate tow by a transport jet nozzle system (TJNS).

4. The stick of claim 1, wherein filter portion comprises a capsule comprising a cough suppressant, and an acetate tow.

5. The stick of claim 1, wherein the cough suppressant is sprayed onto inside or outside of the tube portion.

6. The stick of claim 1, wherein the nicotine dry powder of the capsule portion comprises nicotine or a nicotine salt, amino acid, and an excipient.

7. The stick of claim 6, wherein the nicotine dry powder comprises a nicotine salt, and the nicotine salt comprises nicotine, and a salt comprising one or more selected from the group consisting of lactic acid, tartaric acid, pyruvic acid, and salicylic acid.

8. The stick of claim 6, wherein the nicotine salt comprises nicotine and lactic acid.

9. The stick of claim 6, wherein the amino acid comprises one or more selected from the group consisting of leucine, lysine, valine, arginine, threonine, and methionine.

10. The stick of claim 6, wherein the amino acid comprises leucine, and a content of the leucine is 5 wt% or less based on a total weight of the nicotine dry powder.

11. The stick of claim 6, wherein the excipient comprises one or more of sugar and sugar alcohol,
the sugar comprises one or more selected from the group consisting of lactose, sucrose, trehalose, dextrose, glucose, and maltose, and
the sugar alcohol comprises one or more selected from the group consisting of mannitol, sorbitol, galactitol, maltitol, xylitol, and lactitol.

12. The stick of claim 6, wherein the excipient comprises mannitol.

13. A dry powder inhaler (DPI) comprising the stick for a DPI of claim 1.
